(19) Europäisches Patentamt
European Patent Office
Office européen des brevets

(11) **EP 3 901 126 B1**

(12) **EUROPÄISCHE PATENTSCHRIFT**

(45) Veröffentlichungstag und Bekanntmachung des Hinweises auf die Patenterteilung:
**24.09.2025 Patentblatt 2025/39**

(21) Anmeldenummer: **20020186.1**

(22) Anmeldetag: **20.04.2020**

(51) Internationale Patentklassifikation (IPC):
**C07C 29/151** (2006.01) **C07C 31/04** (2006.01)
**B01J 23/80** (2006.01)

(52) Gemeinsame Patentklassifikation (CPC):
(C-Sets verfügbar)
**C07C 29/1512;** Y02P 20/52; Y02P 20/584 (Forts.)

(54) **VERFAHREN ZUR HERSTELLUNG VON METHANOL**

METHOD FOR PRODUCING METHANOL

PROCÉDÉ DE FABRICATION DE MÉTHANOL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**27.10.2021 Patentblatt 2021/43**

(73) Patentinhaber: **L'AIR LIQUIDE, SOCIÉTÉ ANONYME POUR L'ÉTUDE ET L'EXPLOITATION DES PROCÉDÉS GEORGES CLAUDE**
**75007 Paris (FR)**

(72) Erfinder:
• **SCHUHMANN, Timm**
**60439 Frankfurt am Main (DE)**
• **WURZEL, Thomas**
**60439 Frankfurt am Main (DE)**
• **WILLIAMS, Bryce**
**60439 Frankfurt am Main (DE)**
• **GRONEMANN, Veronika**
**60439 Frankfurt am Main (DE)**
• **OELMANN, Tobias**
**60439 Frankfurt am Main (DE)**
• **HAAG, Stephane**
**60439 Frankfurt am Main (DE)**
• **DO, Nga Thi Quynh**
**60439 Frankfurt am Main (DE)**

(74) Vertreter: **Air Liquide**
**L'Air Liquide S.A.**
**Direction de la Propriété Intellectuelle**
**75, Quai d'Orsay**
**75321 Paris Cedex 07 (FR)**

(56) Entgegenhaltungen:
**WO-A1-2020/048809**

**EP 3 901 126 B1**

(52) Gemeinsame Patentklassifikation (CPC): (Forts.)

C-Sets
**C07C 29/1512, C07C 31/04**

## Beschreibung

### Gebiet der Erfindung

Die Erfindung betrifft ein Verfahren zur Herstellung von Methanol, eine Anlage konfiguriert zur Durchführung des erfindungsgemäßen Verfahrens zur Herstellung von Methanol sowie die Verwendung der Anlage im erfindungsgemäßen Verfahren zur Herstellung von Methanol.

### Stand der Technik

Synthesegase, welche zumindest Kohlenstoffoxide (Kohlenmonoxid und Kohlendioxid) und Wasserstoff enthalten, und aus einer beliebigen kohlenstoffhaltigen Quelle herstellbar sind, können an geeigneten Katalysatoren gemäß den Reaktionen (1) und (2)

$$CO + 2\,H_2 \rightleftharpoons CH_3OH \qquad (1)$$

$$CO_2 + 3\,H_2 \rightleftharpoons CH_3OH + H_2O \qquad (2)$$

zu Methanol umgesetzt werden. Voraussetzung ist, dass die Katalysatorgifte bis zu einem tolerierbaren Schwellenwert aus dem Synthesegas entfernt wurden und die Zusammensetzung des Synthesegases eine geeignete Stöchiometriezahl SN, definiert als

$$SN = \frac{n(H_2) - n(CO_2)}{n(CO) + n(CO_2)} \; ,$$

*mit n in [mol],*
aufweist.

Für die Methanolsynthese eingesetzte Synthesegase werden regelmäßig SN Werte oberhalb von 2,0, oder oberhalb von 2,5 oder sogar oberhalb von 3,0 gefordert werden. Ein SN Wert oberhalb von 2,0 deutet auf einen Wasserstoff-Überschuss hin, ein SN Wert unterhalb von 2,0 auf einen Wasserstoffmangel.

Es entspricht der allgemeinen Auffassung in Fachkreisen, dass die Verwendung von Synthesegaszusammensetzungen mit einer Stöchiometriezahl von nur knapp oberhalb von 2,0 oder gar unterhalb von 2,0 bei der Methanolsynthese zu einer nicht tolerablen Bildung von Nebenprodukten führt.

Ein hohes Ausmaß an Nebenproduktbildung steht für eine niedrige Selektivität im Hinblick auf das Zielprodukt Methanol und führt dadurch zu einer unerwünscht niedrigen Methanol-Ausbeute.

Werden große Mengen an Nebenprodukten gebildet, können diese aus dem primär erhaltenen Rohmethanol gegebenenfalls nicht durch an die Methanol-Herstellung unmittelbar angeschlossene thermische Trennverfahren, wie beispielsweise durch eine Rektifikation, entfernt werden. Ferner steigt der Energieverbrauch beim verwendeten thermischen Trennverfahren und/oder der Verlust an Methanol steigt durch Nebenprodukte, welche aufgrund ähnlicher physikalischer Eigenschaften (wie Siedepunkt, Dampfdruck) schwierig vom Zielprodukt Methanol abzutrennen sind. Die allgemeine Auffassung der Fachkreise ist, dass mit abnehmender Stöchiometriezahl des eingesetzten Synthesegases die Bildung von Nebenprodukten so hoch wird, dass durch Aufarbeitung des Rohmethanols mittels des an die Methanol-Herstellung unmittelbar angeschlossenen thermischen Trennverfahrens kein ausreichend reines Methanol erhalten werden wird, was beispielsweise bei Nebenprodukt-Konzentrationen von mehr als 10.000 ppm (1 Gew.-%) im Rohprodukt der Fall sein kann.

Es besteht somit Bedarf für die Verbesserung bestehender Verfahren.

WO 2020/048809 offenbart ein Verfahren zur Herstellung von Methanol aus einem kohlenstoffhaltigen Einsatzstoff, bei dem in einer Synthesegas-Erzeugungseinheit Synthesegas erzeugt wird, das Synthesegas in einer Methanol-Syntheseeinheit zu Methanol umgesetzt und das erhaltene Reaktionsgemisch stufenweise zur Isolierung des Methanols aufgearbeitet wird. Dabei werden werthaltige Komponenten wie Kohlenmonoxid, Kohlendioxid, Dimethylether und Methan aus den bei der Isolierung des Methanols abgetrennten Strömen mit einem sauerstoffhaltigen Gas verbrannt, das Kohlendioxid des entstandenen Rauchgases in einer Kohlendioxid-Rückgewinnungseinheit abgetrennt und zur Synthesegas-Erzeugungseinheit und/oder zur Methanol-Syntheseeinheit zurückgeführt.

### Beschreibung der Erfindung

Eine Aufgabe der vorliegenden Erfindung besteht darin ein Verfahren zur Herstellung von Methanol bereitzustellen, welches die Nachteile des Standes der Technik zumindest teilweise überwindet.

Eine weitere Aufgabe der vorliegenden Erfindung besteht darin, ein Verfahren zur Herstellung von Methanol bereitzustellen, welches sich durch eine verringerte Bildung von Nebenprodukten auszeichnet.

Eine weitere Aufgabe der vorliegenden Erfindung besteht darin, ein Verfahren zur Herstellung von Methanol bereitzustellen, welches den Einsatz von Synthesegasen mit niedriger Stöchiometriezahl für die Methanolsynthese ermöglicht, und welches sich gleichzeitig durch eine verringerte Bildung von Nebenprodukten auszeichnet.

Eine weitere Aufgabe der vorliegenden Erfindung besteht darin, eine Anlage zur Herstellung von Methanol bereitzustellen, welche zumindest teilweise zumindest eine der vorgenannten Aufgaben löst.

Ein Beitrag zur mindestens teilweisen Erfüllung mindestens einer der obigen Aufgaben wird durch die unabhängigen Ansprüche geleistet. Die abhängigen Ansprüche stellen bevorzugte Ausführungsformen bereit, die zur mindestens teilweisen Erfüllung mindestens einer der Aufgaben beitragen. Bevorzugte Ausgestaltungen von Bestandteilen einer erfindungsgemäßen Kategorie sind, soweit zutreffend, ebenso bevorzugt für gleichnamige oder entsprechende Bestandteile einer jeweils anderen erfindungsgemäßen Kategorie.

Die vorgenannten Aufgaben werden zumindest teilweise gelöst durch ein Verfahren zur Herstellung von Methanol, wobei das Verfahren folgende Prozessschritte umfasst, wobei diese nicht zwingend in der angegebenen Reihenfolge auszuführen sind:

a. Bereitstellen eines Kohlenstoffoxide und Wasserstoff aufweisenden Synthesegases;
b. Leiten des Synthesegases bei erhöhtem Druck und erhöhter Temperatur durch ein Katalysatorbett eines Methanolsynthese-Katalysators zur Umsetzung des Synthesegases zu Methanol, wobei ein Rohmethanol und nicht-reagiertes Synthesegas umfassender Produktstrom erhalten wird;
c. Kühlen des Produktstroms zur Kondensation und Abtrennung von zumindest Methanol und Wasser aufweisendem Rohmethanol aus dem gekühlten Produktstrom;
d. Zurückführen zumindest eines Teils des nicht-reagierten Synthesegases zum Eingang des Katalysatorbetts, wobei das nicht reagierte Synthesegas mit dem Synthesegas zusammengeführt wird, wobei ein gemischtes Synthesegas erhalten wird, und Leiten des gemischten Synthesegases bei erhöhtem Druck und erhöhter Temperatur durch das Katalysatorbett des Methanolsynthese-Katalysators zur Umsetzung des gemischten Synthesegases zu Methanol,

dadurch gekennzeichnet, dass

das gemischte Synthesegas am Eingang des Katalysatorbetts eine Stöchiometriezahl SN von 0,80 bis 2,20 aufweist, wobei gilt

$$SN = \frac{n(H_2) - n(CO_2)}{n(CO) + n(CO_2)} \, ,$$

mit n in [mol],

das Katalysatorbett bei der Umsetzung des gemischten Synthesegases zu Methanol eine maximale Katalysatorbett-Temperatur von ≤ 280 °C aufweist, und das gemischte Synthesegas am Eingang des Katalysatorbettes eine Kohlenmonoxid-Konzentration von 9,0 bis 13,0 Vol.-% aufweist.

Es wurde gefunden, dass die Bildung von Nebenprodukten zurückgedrängt werden kann, wenn

- die maximale Temperatur im Katalysatorbett, also die maximale Katalysatorbett-Temperatur, auf maximal 280 °C begrenzt wird,
- die Stöchiometriezahl des gemischten Synthesegases am Katalysatorbett-Eingang mindestens 0,80 beträgt und
- das gemischte Synthesegas am Eingang des Katalysatorbettes eine Kohlenmonoxid-Konzentration von nicht mehr als 20 Volumen-Prozenten aufweist.

Detaillierte Untersuchungen haben gezeigt, dass das erhaltene Rohmethanol stets eine Konzentration von weniger als 10000 ppm Nebenprodukten aufweist, wenn die vorgenannten, insbesondere die erfindungsgemäß definierten Parameter eingehalten werden. Der in ppm angegebene Nebenproduktgehalt bezieht sich dabei auf die Masse der in Summe gebildeten Nebenprodukte in Relation zur Masse des durch Kühlung aus dem Produktgemisch abgetrennten Rohmethanol, wobei das Rohmethanol aus Methanol ($CH_3OH$), Wasser ($H_2O$) und nicht vermeidbaren Nebenprodukten zusammengesetzt ist. Beispielsweise bedeutet eine Konzentration von 6500 ppm Nebenprodukten, dass 6500 mg Nebenprodukte pro kg Rohmethanol gebildet wurden.

Das erfindungsgemäße Verfahren ist als sogenannter Methanolsynthese-Kreislauf ausgestaltet, das heißt ein Teil des nicht im Katalysatorbett umgesetzten Synthesegases (nicht reagiertes Synthesegas) wird durch Kühlung und dadurch bewirkter Phasentrennung von der kondensierten Rohmethanol-Phase abgetrennt und zum Eingang des Katalysatorbettes zurückgeführt, also recycelt. Dieses zurückgeführte Synthesegas wird mit dem Synthesegas zusammengeführt, wobei das gemischte Synthesegas erhalten wird. Entsprechend wird auch das gemischte Synthesegas zur Umsetzung des Synthesegases zu Methanol bei erhöhtem Druck und erhöhter Temperatur durch das Katalysatorbett geleitet, wobei wiederum ein Rohmethanol und nicht-reagiertes Synthesegas umfassender Produktstrom erhalten wird. Das Synthesegas kann auch als "frisches" Synthesegas, Frischgas oder *make up gas* bezeichnet werden. Das zurückgeführte Synthesegas kann auch als Rückführgas oder re*cycle gas* bezeichnet werden. Das nicht reagierte Synthesegas wird vollständig oder teilweise zum Katalysatorbett-Eingang zurückgeführt und mit dem Synthesegas zusammengeführt. Regelmäßig wird das nicht reagierte Synthesegas nur teilweise zurückgeführt, da üblicherweise ein Teil des nicht reagierten Synthesegases als Spülgas (*purge gas*) von dem nicht reagierten Synthesegas abgezweigt wird. Dadurch soll verhindert werden, dass sich unter den Bedingungen der Methanolsynthese inerte Bestandteile, wie Beispielsweise Methan oder Stickstoff, im Methanolsynthese-Kreislauf anreichern. Weiterhin kann das Spülgas beispielsweise einer Wechseldruckabsorptionsvorrichtung (*pressure swing absorption, PSA*) zugeführt werden, um Wasserstoff von den übrigen Bestandteilen des Spülgases abzutrennen. Der so gewonnene Wasserstoff kann beispielsweise dem Synthesegas zugeführt werden, um dessen Stöchiometriezahl auf einen gewünschten Wert einzustellen.

Es wurde gefunden, dass die Stöchiometriezahl des gemischten Synthesegases am Katalysatorbetteingang einen vergleichsweise niedrigen Minimalwert von nur 0,80 aufweisen muss, damit in Verbindung mit den weiteren definierten Parametern Nebenprodukte in geringfügigem Ausmaß, quantitativ wie oben beschrieben, gebildet werden.

Dabei ist die Stöchiometriezahl des gemischten Synthesegases am Katalysatorbett-Eingang strikt von der Stöchiometriezahl des Synthesegases oder Frischgases zu unterscheiden. Primär erzeugtes Synthesegas weist je nach Herstellverfahren eine Stöchiometriezahl von etwa 1,7 bis 2,2 auf. Durch die Mischung der Ströme des Synthesegases und des zurückgeführten Synthesegases und gegebenenfalls Zuführung von intern oder extern erzeugtem Wasserstoff kann die Stöchiometriezahl des gemischten Synthesegases am Katalysatorbetteingang über einen weitaus größeren Bereich variiert werden.

Eine vergleichsweise niedrige Stöchiometriezahl von 0,80 bedeutet, dass das gemischte Synthesegas arm an Wasserstoff und reich an Kohlenstoffoxiden (Kohlenmonoxid und Kohlendioxid) ist. Dadurch eröffnet sich die Möglichkeit, dass unmodifiziertes Synthesegas, das heißt Synthesegas welches nicht durch eine interne oder externe Quelle mit Wasserstoff angereichert wurde, im erfindungsgemäßen Verfahren verwendet werden kann. Zumindest ist dies dann der Fall, wenn die Kohlenmonoxid-Konzentration im gemischten Synthesegas eine Konzentration von 20 Vol.-% nicht überschreitet und gleichzeitig eine maximale Katalysatorbett-Temperatur von 280 °C eingehalten wird.

Eine bevorzugte Ausführungsform des erfindungsgemäßen Verfahrens ist dadurch gekennzeichnet, dass das Katalysatorbett bei der Umsetzung des gemischten Synthesegases zu Methanol eine maximale Katalysatorbett-Tem-peratur von $\leq$ 265 °C aufweist. Wird die maximale Katalysatorbett-Temperatur derart kontrolliert, dass eine Temperatur von 265 °C nicht überschritten wird, wird die Bildung von unerwünschten Nebenprodukten weiter zurückgedrängt. Untersuchungen haben gezeigt, dass die Menge an unerwünschten Nebenprodukten auf 5000 ppm oder weniger sinkt, wenn die maximale Katalysatorbett-Temperatur auf 265 °C begrenzt wird.

Weiter bevorzugt weist das Katalysatorbett bei der Umsetzung des gemischten Synthesegases zu Methanol eine maximale Katalysatorbett-Temperatur von $\leq$ 250 °C auf. Wird die maximale Katalysatorbett-Temperatur auf 250 °C begrenzt, sinkt die Konzentration an unerwünschten Nebenprodukten auf 3500 ppm oder weniger, wie Untersuchungen gezeigt haben.

Eine bevorzugte Ausführungsform des erfindungsgemäßen Verfahrens ist dadurch gekennzeichnet, dass das Katalysatorbett bei der Umsetzung des gemischten Synthesegases zu Methanol eine maximale Katalysatorbett-Temperatur von 205 °C bis 280 °C aufweist.

Eine weitere bevorzugte Ausführungsform des erfindungsgemäßen Verfahrens ist dadurch gekennzeichnet, dass das Katalysatorbett bei der Umsetzung des gemischten Synthesegases zu Methanol eine maximale Katalysatorbett-Temperatur von 205 °C bis 265 °C aufweist.

Teil der Offenbarung ist ein Beispiel, in welchem das gemischte Synthesegas am Eingang des Katalysatorbetts eine Stöchiometriezahl SN von $\geq$ 2,0 aufweist. Wird die Stöchiometriezahl des gemischten Synthesegases so eingestellt, dass diese am Eingang des Katalysatorbetts für das gemischte Synthesegas einen Wert von 2,0 oder größer annimmt, kann die Bildung von unerwünschten Nebenprodukten im Rohmethanol weiter zurückgedrängt werden. Untersuchungen haben gezeigt, dass die Konzentration an Nebenprodukten im Rohmethanol in diesem Fall stets bei 5000 ppm oder weniger liegt.

Teil der Offenbarung ist ein Beispiel, in welchem das gemischte Synthesegas am Eingang des Katalysatorbetts eine Stöchiometriezahl SN von 0,80 bis 10,0 aufweist.

Gemäß der Erfindung weist das gemischte Synthesegas am Eingang des Katalysatorbetts eine Stöchiometriezahl von 0,80 bis 2,20 auf. Es wurde überraschend gefunden, dass weniger als 10.000 ppm Nebenprodukte gebildet werden, wenn die Stöchiometriezahl des gemischten Synthesegases am Katalysatorbett-Eingang auf 2,20 begrenzt wird und die weiteren erfindungsgemäßen Bedingungen hinsichtlich der minimalen Stöchiometriezahl sowie einer definierten Kohlenmonoxid-Konzentration im gemischten Synthesegas am Katalysatoreingang, und der maximalen Katalysatorbett-Temperatur erfüllt werden. Dabei ist erfindungsgemäß vorgesehen, dass das gemischte Synthesegas am Eingang des Katalysatorbettes eine Kohlenmonoxid-Konzentration von 9,0 bis 13,0 Vol.-% aufweist. Dadurch werden gleichzeitig hohe Wasserstoff-Umsätze von 80 % oder mehr erreicht, bei Einhaltung weiterer Randparameter sogar von 90 % oder mehr. Wasserstoff ist insbesondere bei Synthesegasen, die durch autotherme Reformierung oder durch partielle Oxidation gewonnen werden, das "wertvollste" der Gase in einem Synthesegasgemisch. Auch trifft dies bei der Umsetzung von kohlendioxidreichen Synthesegasen zu Methanol zu. Letztere Technologie gewinnt derzeit immer mehr an Bedeutung. Denn im Zuge der Diskussion über den vom Menschen verursachten Klimawandel und $CO_2$-Bepreisung steigt sowohl das ökologische als auch das wirtschaftliche Interesse daran, Kohlendioxid einer Valorisierung zuzuführen. Somit wird bei den vorgenannten Technologien stets ein hoher Wasserstoff-Umsatz bei der Herstellung von Methanol angestrebt.

Eine bevorzugte Ausführungsform des erfindungsgemäßen Verfahrens ist dadurch gekennzeichnet, dass das Synthesegas eine Stöchiometriezahl SN von 1,0 bis 2,85 aufweist, vorzugsweise eine Stöchiometriezahl SN von 1,0 bis 2,30 aufweist. Das erfindungsgemäße Verfahren eignet sich auch für die Synthesegase mit kleiner Stöchiometriezahl, insbesondere mit einer Stöchiometriezahl von 2,0 oder kleiner. Solche Synthesegase zeichnen sich dadurch aus, dass diese arm an Wasserstoff und/oder reich an Kohlendioxid im Vergleich zu Kohlenmonoxid sind. Das erfindungsgemäße Verfahren eignet sich somit auch für nicht modifizierte Synthesegase, die nicht auf eine interne oder externe Wasserstoffquelle angewiesen sind, sowie für Synthesegase, die in Bezug auf die Kohlenstoffoxide hauptsächlich oder sogar ausschließlich Kohlendioxid aufweisen.

Gemäß einer Ausführungsform des erfindungsgemäßen Verfahrens liegt das Verhältnis von nicht-reagiertem, zurückgeführtem Synthesegas zu Synthesegas im gemischten Synthesegas, definiert als Rezirkulationsrate RR, wobei gilt

$$RR = \frac{Volumenstrom\ (zur\ddot{u}ckgef\ddot{u}hrtes\ Synthesegas)}{Volumenstrom\ (Synthesegas)},$$

bei 2,0 bis 4,5. Der Volumenstrom des zurückgeführten Synthesegases beträgt in diesem Fall mindestens das Doppelte bis zum viereinhalbfachen des Volumenstroms des (frischen) Synthesegases.

Eine bevorzugte Ausführungsform des erfindungsgemäßen Verfahrens ist dadurch gekennzeichnet, dass das gemischte Synthesegas am Eingang des Katalysatorbettes eine Kohlendioxid-Konzentration von $\geq$ 20,0 Vol.-% aufweist. Es wurde überraschend gefunden, dass Synthesegase mit sehr hohem Kohlendioxid-Gehalt von 20,0 Volumenprozenten oder mehr zur Bildung von sehr wenig unerwünschten Nebenprodukten führen, sofern die weiteren erfindungsgemäßen Bedingungen eingehalten werden. Untersuchungen haben in diesem Fall gezeigt, dass die Konzentration an unerwünschten Nebenprodukten in Rohmethanol stets unter 1000 ppm liegt. Somit eignet sich das erfindungsgemäße Verfahren insbesondere für Synthesegase, die reich an Kohlendioxid und arm an Kohlenmonoxid sind. Vorzugsweise weist das gemischte Synthesegas gemäß dieser Ausführungsform eine Kohlenmonoxid-Konzentration von weniger als 5 Vol.-% auf, oder weniger als 3 Vol.-%, oder weniger als 1 Vol.-%. Dabei kann es sich beispielsweise um ein Synthesegas handeln, welchem eine größere Menge eines Abgases aus einer Verbrennungsanlage zugemischt wurde.

Eine bevorzugte Ausführungsform des erfindungsgemäßen Verfahrens ist dadurch gekennzeichnet, dass das Katalysatorbett auf mehrere in Serie angeordnete Katalysatorbettstufen aufgeteilt ist, wobei Schritt c) nach jeder der Katalysatorbettstufen durchgeführt wird. Gemäß dieser auch als Multireaktor-Konzept oder Multireaktorstufen-Konzept bezeichneten Ausführungsform findet nach jeder der Katalysatorbettstufen eine Kondensation von in der Katalysatorbettstufe gebildetem Rohmethanol statt, welches dementsprechend an mehreren Stellen aus dem Verfahren ausgeschleust wird. Je mehr Reaktorstufen oder Katalysatorbettstufen zum Einsatz kommen, desto weniger nicht reagiertes Synthesegas muss zum Eingang der ersten Katalysatorbett-Stufe zurückgeführt werden. Die Kohlenstoffausbeute kann durch eine Mehrzahl von Katalysatorbettstufen verbessert werden.

Eine bevorzugte Ausführungsform des erfindungsgemäßen Verfahrens ist dadurch gekennzeichnet, dass Schritt b) bei einem Druck von 30 bis 120 bar durchgeführt wird, vorzugsweise bei einem Druck von 40 bis 90 bar durchgeführt wird. Die angegebenen Druckbereiche entsprechen den üblichen, in modernen Niederdruckverfahren bei der Methanol-Herstellung eingesetzten Drücken.

Eine bevorzugte Ausführungsform des erfindungsgemäßen Verfahrens ist dadurch gekennzeichnet, dass Schritt b) bei einer Raumgeschwindigkeit von 2000 bis 16.000 $Nm^3/(m^3h)$ durchgeführt wird. Die angegebenen Raumgeschwindigkeiten entsprechen Verweilzeiten der Reaktanten im Katalysatorbett, die zu besonders hohen Kohlenstoff-Konversionsraten führen.

Eine bevorzugte Ausführungsform des erfindungsgemäßen Verfahrens ist dadurch gekennzeichnet, dass ein Teil des nicht-reagierten Synthesegases als Spülgas entnommen wird. Dadurch wird verhindert, dass sich größere Mengen an unter den Bedingungen der Methanol-Synthese inerten Bestandteilen im Methanolsynthese-Kreislauf anreichern.

Eine bevorzugte Ausführungsform des erfindungsgemäßen Verfahrens ist dadurch gekennzeichnet, dass die Umsetzung des Synthesegases zu Methanol im Katalysatorbett bei einer Kühltemperatur des verwendeten Kühlmediums von 190 °C bis 250 °C erfolgt. Durch die Wahl der entsprechenden Temperatur des Kühlmediums oder Kühlmittels, üblicherweise unter Druck stehendes siedendes Wasser, kann die maximale Katalysatorbett-Temperatur entsprechend eingestellt werden.

Teil der Offenbarung ist auch eine Anlage zur Herstellung von Methanol, konfiguriert zur Durchführung des erfindungsgemäßen Verfahrens nach einer der vorgenannten Ausführungsformen.

Ferner ist Teil der Offenbarung die Verwendung der vorgenannten Anlage in einem Verfahren nach einer der vorgenannten Ausführungsformen zur Herstellung von Methanol.

Katalysator, Katalysatorbett

Das Katalysatorbett ist ein Festbett auf Basis eines dem Fachmann bekannten Methanolsynthese-Katalysators. Das Festbett des Katalysatorbetts ist in einem Beispiel als Schüttung von losen Partikeln, zum Beispiel Pellets, beispielsweise in Tabletten- oder Zylinderform, ausgestaltet. In einem weiteren Beispiel ist das Festbett des Katalysatorbetts als strukturierter Katalysator, beispielsweise mit poröser monolithischer Struktur, ausgestaltet.

In Zusammenhang mit Gegenständen der Erfindung wird unter dem Eingang des Katalysatorbetts ein Bereich verstanden, der stromaufwärts zum Katalysatorbett liegt, und in dem noch keine Umsetzung von Synthesegas und/oder gemischtem Synthesegas zu Rohmethanol stattgefunden hat. Vorzugsweise wird unter dem Eingang des Katalysatorbetts ein Bereich verstanden, der unmittelbar vor dem Katalysatorbett liegt. Mit anderen Worten, unmittelbar nach dem Eingang des Katalysatorbetts tritt das Synthesegas in das Katalysatorbett ein.

Bei dem Methanolsynthese-Katalysator kann es sich um einen beliebigen dem Fachmann bekannten Katalysator handeln. In einem Beispiel handelt es sich um einen Katalysator auf Basis von Kupfer als katalytisch aktiver Spezies. Beispiele für weitere Bestandteile, insbesondere eines auf Kupfer basierenden Katalysators, sind Zinkoxid, Aluminiumoxid ("Alumina"), Chromoxid, Titanoxid, Zirkoniumoxid (Zirkon) und Magnesiumoxid. Ein Beispiel für einen häufig eingesetzten Katalysator ist ein Katalysator welcher zumindest Kupfer, ZnO und $Al_2O_3$ aufweist. Katalysatoren auf Kupferbasis sind beispielsweise über einen Temperaturbereich von 180 °C bis 300 °C verwendbar.

Maximale Katalysatorbett-Temperatur

Tritt ein Synthesegasgemisch in einen gekühlten Methanolsynthese-Reaktor ein, ist die Temperatur des Synthesegases meist zunächst niedriger als die Temperatur des verwendeten Kühlmittels.

Das verwendete Kühlmittel ist entweder ein gasförmiges oder flüssiges Kühlmittel. Ein Beispiel für ein gasförmiges Kühlmittel ist eingesetztes Synthesegas und/oder Rückführgas, das durch die Kühlung der Prozessgase vorgewärmt wird. Ein Beispiel für ein flüssiges Kühlmittel ist unter erhöhtem Druck stehendes siedendes Wasser, welches durch die Kühlung des Reaktionsgemisches verdampft und als Exportdampf oder verfahrensintern als Heizdampf oder Prozessdampf weiterverwendet werden kann.

Ein erster Teil des Katalysatorbetts dient der Erwärmung des Synthesegases, wobei Wärme vom Kühlmittel auf das Synthesegas und den Katalysator übertragen wird. In Zuge dessen beginnt allmählich die Reaktion zur Methanolbildung, bei der aufgrund des exothermen Charakters der Reaktion Wärme erzeugt und die Temperatur sowohl des Katalysators als auch des Gasgemisches (Synthesgas und gasförmiges Methanol/Wasser, sowie nicht-reagiertes Synthesegas) erhöht wird. Im weiteren Verlauf der Reaktion entspricht die Temperatur des Katalysatorbetts und des Gasgemischs in etwa der Temperatur des Kühlmittels.

In einem zweiten Teil des Katalysatorbetts schreitet die Reaktion weiter fort, wobei weiter Wärme erzeugt und das Katalysatorbett und das Gasgemisch weiter erwärmt werden. Die Geschwindigkeit der Wärmeerzeugung in diesem zweiten Teil des Katalysatorbettes ist schneller als die Wärmeübertragung vom Kühlmittel, so dass die Temperaturen des Gasgemischs und des Katalysatorbetts über die Temperatur des Kühlmittels steigen. Dabei erwärmt die bei der Reaktion erzeugte Wärme zunächst den festen Katalysator. Anschließend erfolgt Wärmeübertragung vom Katalysator auf das Gasgemisch, um den Katalysator zu kühlen. Anschließend überträgt das Gasgemisch die Wärme auf das im Reaktor verwendete Kühlmittel. Eine weitere Art der Wärmeübertragung ist Wärmekonvektion vom festen Katalysator auf die Reaktoreinbauten. Die Temperatur steigt in diesem Teil des Katalysatorbetts weiter über die des Kühlmittels. Im Verlauf der Reaktion werden die Reaktanten weiter verbraucht und mehr und mehr Rohmethanol wird erzeugt. Da es sich bei der katalytischen Methanolsynthese um eine Gleichgewichtsreaktion handelt, nähert sich die Reaktionsgeschwindigkeit und damit auch die Wärmeproduktionsrate bei Erreichen der Gleichgewichtskonzentration von Reaktanten und Produkten einem Grenzwert.

In einem dritten Teil des Katalysatorbetts verlangsamt sich die Geschwindigkeit der Wärmeproduktion, da sich die Reaktion den Gleichgewichtsbedingungen nähert. Die Wärmeübertragung vom Katalysator auf das Gasgemisch und letzlich auf das Kühlsystem setzt sich dennoch fort und ermöglicht es, die Temperatur des Katalysatorbetts wieder zu senken.

In einem letzten, vierten Teil des Katalysatorbetts befindet sich die Reaktion im Gleichgewicht, ohne dass eine nennenswerte Wärmeproduktion stattfindet. In diesem Teil des Katalysatorbetts sinkt die Temperatur weiter in Richtung der Kühlmitteltemperatur.

Die maximale Katalysatorbett-Temperatur tritt demnach, wie vorgehend beschrieben, zwischen dem zweiten und dritten Teil des Katalysatorbetts auf. In diesem Temperaturmaximum ist die Geschwindigkeit der Bildung der Reaktionswärme in etwa mit der Geschwindigkeit der Wärmeübertragung im Gleichgewicht, so dass die Temperatur an dieser Stelle des Katalysatorbetts weder steigt noch sinkt.

In der Praxis kann die maximale Katalysatorbett-Temperatur mit bekannten Methoden unmittelbar gemessen werden. Im Labor- oder Technikumsmaßstab kann beispielsweise eine Tauchhülse innerhalb des Katalysatorbetts positioniert und ein Thermoelement manuell an verschiedene Positionen in der Tauchhülse bewegt werden, um die Temperatur in longitudinaler Richtung entlang des Katalysatorbetts zu messen. Das Profil der Katalysatorbett-Temperatur kann auf diese Weise in einem Reaktorrohr ermittelt werden, wobei der Scheitelpunkt des Profils der maximalen Katalysatorbett-Temperatur entspricht.

Im Industriemaßstab kann beispielsweise ein Mehrpunkt-Thermoelement verwendet werden, um die Temperatur entlang des Katalysatorbetts gleichzeitig an mehreren Messpositionen zu überwachen. Eine weitere Alternative für die Anwendung im Industriemaßstab ist die Verwendung mehrerer Thermoelemente, die in verschiedenen Reaktorkanälen und in unterschiedlichen Höhen innerhalb des Katalysatorbetts positioniert sind. Auf diese Weise kann ein vollständiges Bild über die Temperaturverteilung im Katalysatorbett im gesamten Reaktor erzeugt werden.

Es ist teuer und aufwändig, solche Messeinrichtungen in industriellen Reaktoren zu verwenden, um die maximale Katalysatorbetttemperatur unmittelbar zu messen. Daher kann in der Design-Phase einer Anlage, aber auch als Routine-Reaktorüberwachungswerkzeug, eine Simulation der Reaktorverhältnisse im Betriebszustand eingesetzt werden, um die Reaktionsgeschwindigkeit entsprechend der gemessenen Reaktionskinetik und der gegebenen Gaszusammensetzung zu modellieren. Eine Reihe von Referenzen für die Kinetik der Methanolreaktion stehen dem Fachmann dabei zur Verfügung. Beispiele sind in der folgenden Tabelle angegeben.

| | |
|---|---|
| Coteron, A; Hayhurst, AN | Kinetics of the synthesis of methanol from CO + $H_2$ and CO + $CO_2$ + $H_2$ over copper-based amorphous catalysts. In: Chemical Engineering Science 49 (1994), Nr. 2, S. 209-221 |
| Graaf, GH; Sijtsema, PJJM ; Stamhuis, EJ ; Joosten, GEH | Chemical equilibria in methanol synthesis. In: Chemical Engineering Science 41 (1986), Nr. 11, S. 2883-2890 |
| Graaf, GH; Stamhuis, EJ ; Beenackers, AACM: | Kinetics of low-pressure methanol synthesis. In: Chemical Engineering Science 43 (1988), Nr. 12, S. 3185-3195 |
| Skrzypek, J ; Lachowska, M ; Moroz, H: | Kinetics of methanol synthesis over commercial copper/zinc oxide/alumina catalysts. In: Chemical Engineering Science 46 (1991), Nr. 11, S. 2809-2813 |

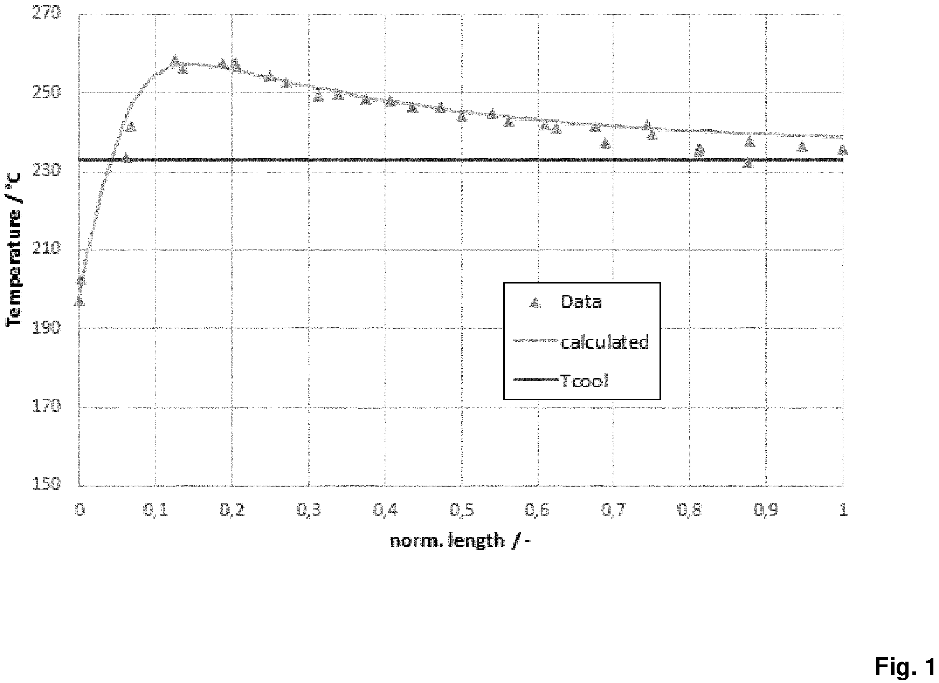

Figur 1 zeigt eine Computersimulation ("calculated") im Vergleich mit experimentell ermittelten Daten ("data") eines kommerziellen Rohrreaktors für die Methanolherstellung. Aufgetragen ist die simulierte und gemessene Katalysatorbett-Temperatur über die normierte Länge des Rohrreaktors. Gezeigt wird ferner die Kühlmitteltemperatur ("Tcool"), welche im gezeigten Fall 232 °C beträgt. Aus der Abbildung der Figur 1 sind die vier Temperatur-Bereiche des Katalysatorbetts entsprechend der obigen Erläuterungen erkennbar. Die maximale Katalysatorbett-Temperatur liegt in diesem Beispiel bei etwa 254 °C. Auch zeigt das Beispiel, dass es möglich ist die tatsächlichen Verhältnisse im Reaktor auf Basis einer Computersimulation mit sehr großer Genauigkeit vorauszusagen.

Darüber hinaus kann, entsprechend den Modellvorstellungen wie oben erläutert, der Wärme- und Massentransfer innerhalb des Katalysatorbetts, vom Katalysatorbett in die Gasphase und schließlich die Wärmeübergang zu den Kühlflächen innerhalb des Reaktors modelliert werden. Die folgende Tabelle enthält eine Sammlung von Referenzen auf typische Modelle und Korrelationen, die für die oben genannten Prozesse verwendet werden. Solche Modelle können von einem Fachmann erstellt werden und erfordern einige zusätzliche bekannte oder leicht messbare Parameter wie die physikalischen Eigenschaften des Katalysators, Druckabfallkorrelationen und Zustandsgleichungen für die Gasmischung.

| Eisfeld, B.; Schnitzlein, K. | The influence of confining walls on the pressure drop in packed beds. Chemical Engineering Science, 56(14):4321 - 4329, 2001. |
|---|---|
| Zhavoronkov, N.M., Aerov, M.E., Umnik, N.N.. | Hydraulic resistance and packing density of a disperse layer. Zh. Fiz. Khim, 23(1):342-360, 1949. |
| Jeschar, R. | Druckverlust in Mehrkornschüttungen aus Kugeln. Archiv für das Eisenhüttenwesen, 35(2):91-108, 1964. |
| Poling, B.E., et al. | The properties of gases and liquids, Volume 5, McGraw-Hill, New York, 2001 |
| Ergun, S. | Fluid flow through packed columns. Chem. Eng. Prog., 48:89-94, 1952. |
| Soave, G | Equilibrium constants from a modified Redlich-Kwong equation of state. In: Chemical Engineering Science 27 (1972), Nr. 6, S. 1197-1203 |

Die maximale Katalysatorbett-Temperatur kann auf verschiedene Weise beeinflusst und kontrolliert werden, um den Betriebspunkt des Reaktors so einzustellen, dass er sich innerhalb eines vorbestimmten Prozessfensters befindet.

Während der Auslegungsphase des Reaktors kann die maximale Katalysatorbett-Temperatur, wie oben gezeigt, durch eine Simulation vorhergesagt werden. Um die maximale Katalysatorbett-Temperatur zu beeinflussen, können eine Reihe von einem Fachmann bekannte Reaktoreigenschaften eingestellt werden. Beispielsweise kann die Kühlmitteltemperatur geändert werden, um die maximale Katalysatorbett-Temperatur zu erhöhen oder zu senken. Auch können die Dimensionen des Katalysatorbetts zur Verbesserung der Wärmeübertragungseigenschaften verändert werden. Ein Beispiel hierfür ist die Verwendung einer Mehrzahl von Rohren mit kleinerem Durchmesser in einem Rohrreaktor zur Verbesserung der Wärmeübertragung, wodurch die maximale Katalysatorbett-Temperatur gesenkt wird. Alternativ kann der Abstand zwischen den Kühlplatten verringert werden, um die maximale Katalysatorbett-Temperatur zu senken. Ferner kann der Gas-Volumenstrom erhöht werden, um die maximale Katalysatorbett-Temperatur zu senken. Weiterhin kann die Gaszusammensetzung so verändert werden, dass die Reaktivität verringert wird und die maximale Katalysatorbett-Temperatur entsprechend sinkt. Dies kann über die Synthesegaszusammensetzung erfolgen oder über die Zugabe von Wasserdampf und/oder Methanol. Eine weitere Möglichkeit in der Auslegungsphase ist die Neuformulierung des Katalysators zur Einstellung der Katalysator-Aktivität. Dies kann durch Änderung der physikalischen Eigenschaften des Katalysators erfolgen, indem beispielsweise Katalysatorpellets unterschiedlicher Größe bei gleicher Zusammensetzung eingesetzt werden, oder durch Verdünnung des aktiven Katalysatormaterials mit unterschiedlichen Mengen an inertem Trägermaterial. Die Katalysatoraktivität kann auch chemisch verändert werden, indem mehr oder weniger aktive Katalysatormaterialien verwendet werden, die dem Fachmann bekannt sind.

Der Methanol-Reaktor ist Teil eines Synthesekreislaufs mit zumindest teilweiser Rückführung des nicht-reagierten Synthesegases. Dadurch kann die maximale Katalysatorbett-Temperatur auch über die Rezirkulationsrate RR gesteuert werden. Insbesondere mit steigender Stöchiometriezahl SN führt eine größere Rezirkulationsrate zu einer Senkung der maximalen Katalysatortemperatur, da das Gasgemisch weniger reaktives Gas aufweist, das gleichzeitig für eine verbesserte Wärmeübertragung sorgt. Ferner ist es zur Steuerung der maximalen Katalysatortemperatur möglich, die Kühlmitteltemperatur über einen engen Bereich durch Anpassung des Drucks der Kühlmitteldampftrommel einzustellen. Im dem Fall dass bestimmte Einschränkungen die Einstellung der maximalen Katalysatortemperatur verhindern, ist es immer noch möglich, während eines Stillstands einer Anlage den Katalysator durch einen oder mehrere Katalysatoren zu ersetzen, die ein anderes Aktivitätsprofil aufweisen, wodurch die maximale Katalysatorbett-Temperatur als Funktion der Katalysatoraktivität eingestellt werden kann.

Nebenprodukte

Das bei der katalytischen Reaktion von Synthesegas und/oder gemischtem Synthesegas zu Methanol gebildete Rohmethanol enthält Wasser und darüber hinaus nicht vermeidbare Nebenprodukte. Die am häufigsten vorkommenden Nebenproduktgruppen sind

- Kohlenwasserstoffe, welche häufig auch als Wachse bezeichnet werden, beispielsweise Hexan, Heptan,
- Ether, insbesondere Dimethylether, sowie Ether mit längeren Kohlenstoff-Ketten,
- Ester, beispielsweise Methylformiat und Ethylformiat,
- Ketone, beispielsweise Aceton, Methylethylketon, sowie
- höhere Alkohole, beispielsweise Ethanol.

Die Gesamtmenge der Nebenprodukte im Rohmethanol ist beispielsweise die Gesamtmenge aller oben genannten Einzelgruppen.

Eine detaillierte Diskussion der Nebenproduktklassen bei der Methanol-Herstellung findet sich in G.C.Chinchen et al., Appl. Catal. 36 (1988) 1-65.

Erhöhter Druck

Das Synthesegas wird für die katalytische Reaktion zu Methanol bei erhöhtem Druck, auch Reaktionsdruck, durch das Katalysatorbett geleitet. Der Reaktionsdruck ist der für die katalytische Reaktion der Bestandteile des Synthesegases und/oder gemischten Synthesegases zu Methanol vorherrschende und erforderliche Druck, um das Synthesegas und/oder gemischte Synthesegas zu Methanol umzusetzen. In einem Beispiel beträgt der Reaktionsdruck im Katalysatorbett 30 bis 120 bar, vorzugsweise 40 bis 90 bar, besonders bevorzugt 75 bis 90 bar und weiter bevorzugt 75 bis 85 bar.

Synthesegas

Das Synthesegas weist zumindest Wasserstoff ($H_2$) und Kohlenstoffoxide auf. Unter dem Begriff "Kohlenstoffoxide" werden die Verbindungen Kohlenmonoxid (CO) sowie Kohlendioxid ($CO_2$) subsummiert. In Bezug auf die Gesamtmenge der Kohlenstoffoxide weist das Synthesegas vorzugsweise einen Kohlenmonoxid-Anteil von wenigstens 20 Vol.-% auf. Vorzugsweise ist das Synthesegas hochkohlenmonoxidhaltig. In einem Beispiel weist das Synthesegas in Bezug auf die Kohlenstoffoxide wenigstens 50 Vol.-% Kohlenmonoxid auf, oder zumindest 70 Vol.-%, oder zumindest 90 Vol.-%, oder zumindest 95 Vol.-%, oder zumindest 99 Vol.-%. In einem Beispiel weist das Synthesegas in Bezug auf die Kohlenstoffoxide nahezu ausschließlich Kohlenmonoxid auf, wobei Kohlendioxid in diesem Fall nur noch in Spuren vorhanden ist. Ein solches Synthesegas kann beispielsweise durch Behandlung eines Roh-Synthesegases in einer Methanol-Wäsche erhalten werden. Im Rahmen einer Methanol-Wäsche oder anderer geeigneter Gaswäscheverfahren kann Kohlendioxid praktisch vollständig entfernt werden. Ein besonders hierfür geeignetes Verfahren ist das selektive Rectisol®-Verfahren.

Das erfindungsgemäße Verfahren eignet sich darüber hinaus auch für hochkohlendioxidhaltige Synthesegase, die in Bezug auf die Kohlenstoffoxide einen Kohlendioxidgehalt von mindestens 50 Vol.-%, oder mindestens 75 Vol.-%, oder mindestens 90 Vol.-% Kohlendioxid enthalten. Dadurch kann auch Kohlenstoff aus einer Kohlendioxid-Quelle der Methanolsynthese zugänglich gemacht werden, was im Zuge der Diskussion über den vom Menschen verursachten Klimawandel zunehmend an Bedeutung gewinnt.

Das Synthesegas kann aus jeder dem Fachmann bekannten Quelle stammen. Beispiele sind Dampfreformierung, partielle Oxidation oder autotherme Reformierung von Erdgas oder anderen geeigneten Kohlenstoffquellen, sowie Vergasung von Kohle oder anderen Festbrennstoffen wie Biomasse oder kommunaler Müll. Kohlendioxid des Synthesegases kann auch aus einer Abgasquelle stammen, beispielsweise einer Müllverbrennungsanlage. Der Wasserstoff des Synthesegases kann auch aus einer Wasserelektrolyse-Anlage stammen, wobei der elektrische Strom für diese Anlage vorzugsweise durch eine regenerative Energiequelle wie Wasserkraft, Windkraft oder Photovoltaik erzeugt wurde.

Das Synthesegas kann, unabhängig von der Quelle aus der es stammt, bei einer Temperatur zwischen 400 °C und 1200 °C und/oder bei einem Druck zwischen 10 und 60 bar erzeugt werden. Das Synthesegas kann außer den vorgenannten Bestandteilen auch unterschiedliche Mengen inerter Bestandteile wie Methan oder Stickstoff enthalten. Unter inerten Bestanteilen sind dabei insbesondere unter den Bedingungen der Methanolsynthese inerte Bestandteile zu verstehen, das heißt Bestandteile welche unter den Bedingungen der Methanolsynthese nicht zu Methanol oder (unerwünschten) Nebenprodukten umgesetzt werden.

Das Synthesegas wird üblicherweise unter den Taupunkt von Dampf zur Auskondensation von Wasser abgekühlt, bevor es im erfindungsgemäßen Verfahren verwendet wird. Insbesondere wird das Synthesegas auf unter 100 °C, vorzugsweise auf unter 60 °C, und weiter bevorzugt auf 40 °C oder weniger abgekühlt, um Wasser nach Kondensation vom Synthesegas abzutrennen. Das Synthesegas ist somit insbesondere frei oder weitgehend frei von Wasser.

Wasserstoff-Umsatz, Kohlenstoff-Umsatz

Der Wasserstoff-Umsatz beziehungsweise Kohlenstoff-Umsatz ist der Anteil des im frischen Synthesegas enthaltenen Wasserstoff beziehungsweise in Kohlenmonoxid oder Kohlendioxid enthaltene Kohlenstoff, der letztlich zu Rohmethanol umgesetzt wird. Die Summe des aus Kohlenmonoxid und Kohlendioxid umgesetzten Kohlenstoffs ist der Gesamtkohlenstoffumsatz. Der Umsatz wird durch die Menge an beispielsweise abgezweigtem Spülgas oder im Rohmethanol gelösten Gasen gemindert. Gelöste Gase sind diejenigen Bestandteile des Synthesegases, die bei der Kondensation des Rohmethanols im Rohmethanol gelöst bleiben. Sie können beispielsweise bei einer zweistufigen Kondensation mit einem Hochdruck- und einem Niederdruckabscheider aus dem Rohmethanol im Niederdruckabscheider ausgegast werden. Gemäß diesem Beispiel ergibt sich als Formel für die Berechung des Umsatzes

$$X_i = 1 - \left( \frac{n(Sp\ddot{u}lgas) + n(gel\ddot{o}ste\ Gase)}{n\ (Frischgas)} \right)$$

mit dem Umsatz $X_i$ des Bestandteils i in mol/mol und den Stoffmengen des jeweiligen Bestandteils (Wasserstoff, Kohlenmonoxid oder Kohlendioxid) im Spülgas (n(Spülgas), gelösten Gasen (n(gelöste Gase) und Frischgas (n(Frischgas)) in mol.

Methanolsynthese-Kreislauf, Rezirkulationsrate

Da es sich bei der Methanol-Bildung aus Kohlenstoffoxiden und Wasserstoff um eine Gleichgewichtsreaktion handelt, wird nicht-reagiertes Synthesegas als Rückführgas (auch Recycle-Gas) zum Eingang des Katalysatorbetts zurückgeführt, um möglichst hohe Kohlenstoff- und Wasserstoff-Umsätze zu erzielen. In diesem Fall spricht man im Gegensatz zu sogenannten *once-through* Verfahren von einem Synthesekreislauf. An üblichen Kupfer/Zinkoxid/Aluminiumoxid basierten Katalysatoren können so unter optimalen Bedingungen Kohlenstoff-Umsätze von 99 % und mehr erzielt werden, das heißt 99 % oder mehr des eingesetzten Kohlenstoffs, sei es als Kohlenmonoxid oder Kohlendioxid, findet sich anschließend in Methanol gebunden wieder. Das Verhältnis von zurückgeführtem, nicht-reagiertem Synthesegas (Rückführgas) zu frisch eingesetztem Synthesegas wird auch als Rezirkulationsrate RR bezeichnet, die definiert ist als

$$R = \frac{Volumenstrom\ (R\ddot{u}ckf\ddot{u}hrgas)}{Volumenstrom\ (Synthesegas)},$$

wobei Werte bis zu 4 nicht ungewöhnlich sind. Das heißt die Menge des zurückgeführten, nicht-reagierten Synthesegases kann bis zum 4-fachen des eingesetzten (frischen) Synthesegases betragen.

**Ausführungsbeispiele**

Die Erfindung wird im Folgenden durch Beispiele näher erläutert, ohne den Gegenstand der Erfindung dadurch zu beschränken.
Es zeigt

Figur 1          ein messtechnisch und durch eine Simulation ermitteltes Temperaturprofil des Katalysatorbetts über die Länge eines Methanol-Rohrreaktors, welches die maximale Katalysatorbett-Temperatur zeigt,

Figur 2          ein vereinfachtes schematisches Verfahrensfließbild einer Versuchsanlage zur Durchführung des erfindungsgemäßen Verfahrens gemäß den in den Figuren 3a und 3b dargestellten Zahlenbeispielen,

Figur 3a und 3b     eine tabellarische Aufstellung der mit der Versuchsanlage gemäß Figur 2 erzielten Ergebnisse.

Figur 1 zeigt einen typischen Temperaturverlauf entlang des Katalysatorbetts eines Methanolsynthese-Reaktors, wie vorstehend erläutert.

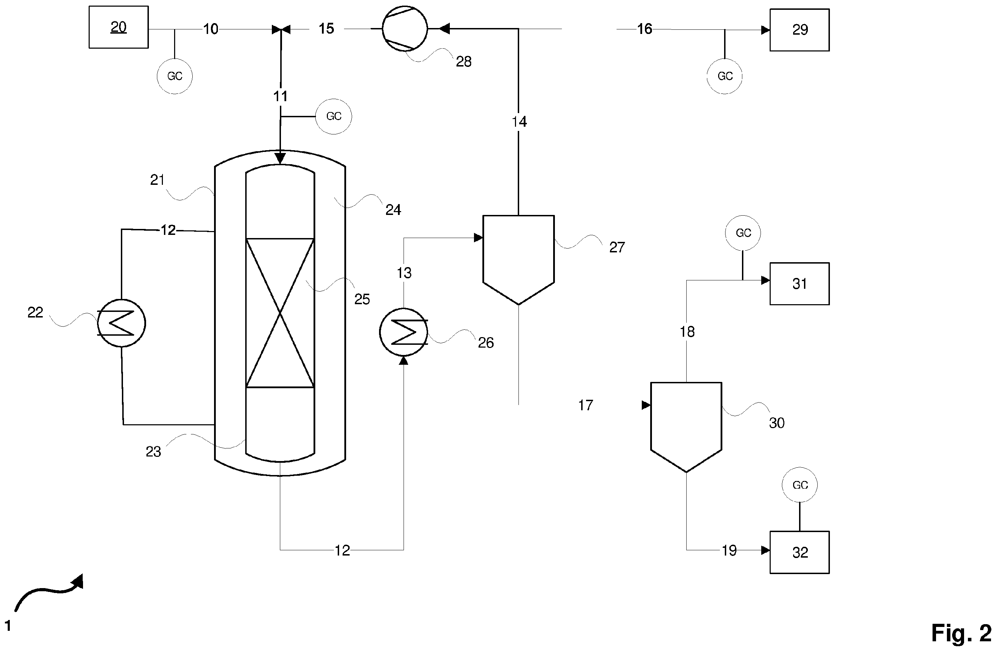

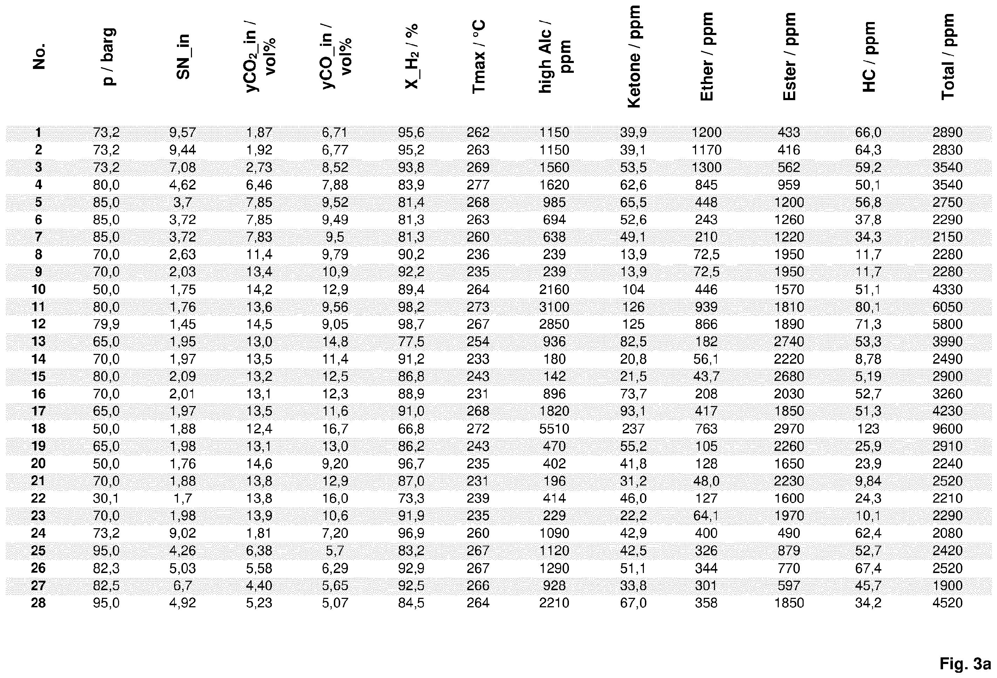

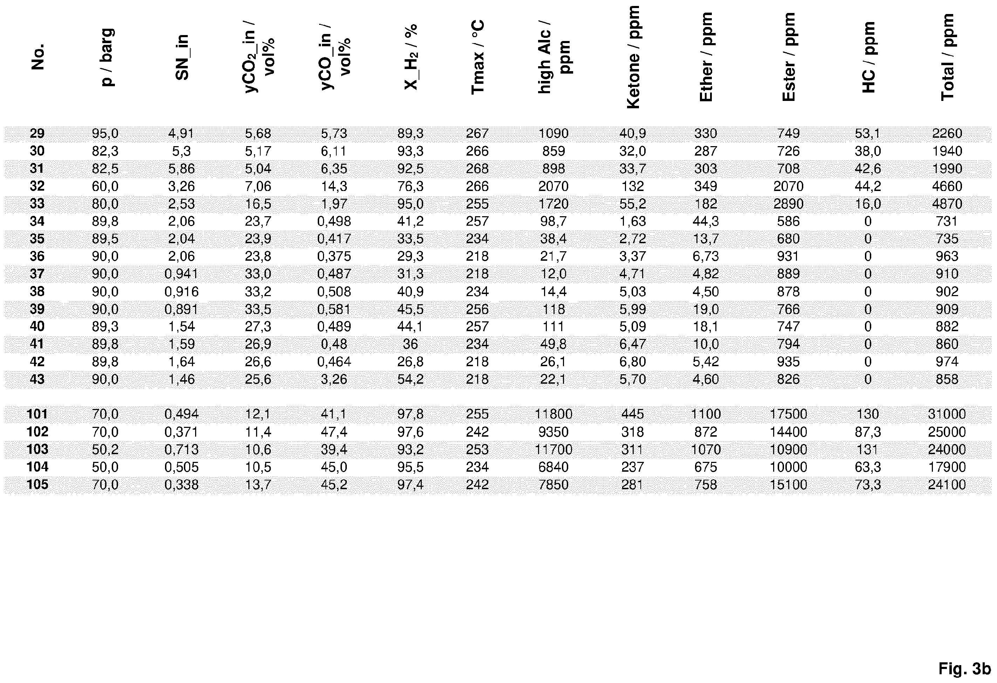

Figur 2 zeigt das Verfahrensschema einer Versuchsanlage 1 zur Methanolsynthese, welche zur Charakterisierung des erfindungsgemäßen Verfahrens und zur Ermittlung der Ergebnisse gemäß der tabellarischen Aufstellung der Figuren 3a und 3b verwendet wurde.
In einer Mischstation 20 wird ein durch Dampf vorgeheiztes Synthesegas (Heizung nicht gezeigt), bestehend aus Wasserstoff, Kohlenmonoxid und Kohlendioxid aus den entsprechenden in technischer Qualität bereitgestellten Reingasen erzeugt und bei erhöhtem Druck (p in barg) über die Leitungen 10 und 11 in den wassergekühlten Reaktor 21 eingeschleust.
Die Zusammensetzung des Synthesegases wird entsprechend den Beispielen 1 bis 43 und den nicht erfindungsgemäßen Beispielen 101 bis 105 (siehe Figuren 3a und 3b) so variiert, dass für das frische Synthesegas in Leitung 10 eine Stöchiometriezahl (SN_MUG) zwischen 0,97 und 2,17 resultiert.
Wassergekühlter Reaktor 21 wird über Wärmeaustauscher 22 und einen mit einem Dampferzeuger (nicht gezeigt) gekoppelten Wasserkreislauf 12 mit unter erhöhtem Druck stehendem, siedendem Wasser gekühlt. Das Kühlwasser umspült ein Reaktionsrohr 23 von Reaktor 21 in Kühlmantel 24. Das Reaktionsrohr 23 (Außendurchmesser x Wanddicke = 33,7 mm x 4,05 mm; Volumen = 3 dm³) weist ein Katalysatorbett 25 auf, welches mit zylinderförmigen Katalysator-Pellets (Clariant Megamax 800, 6x4 mm) auf Basis von $Cu/ZnO/Al_2O_3$ befüllt ist. Die Katalysatorbett-Höhe beträgt 501 cm. Die Kühlmanteltemperatur (T(cool)) beziehungsweise die Temperatur des vorgeheizten Synthesegases wird entsprechend den Beispielen der Figuren 3a und 3b so variiert, dass unterschiedliche maximale Katalysatorbett-Temperaturen (Tmax) resultieren. Das Temperaturprofil innerhalb des Katalysatorbetts 25, welches auch die maximale Katalysatorbett-Temperatur beinhaltet, wird entsprechend der oben beschriebenen Methode mit Hilfe einer Tauchhülse und einem Multipunkt-Thermoelement (nicht gezeigt) ermittelt, um die Temperaturen verschiedener Positionen innerhalb des Katalysatorbetts 25 zu erfassen.

Das im Reaktionsrohr 23 des Reaktors 21 erzeugte Rohmethanol, welches Methanol, Wasser sowie unvermeidbare Verunreinigungen enthält, wird über Leitung 12 abgezogen, in Wärmeaustauscher 26 vorgekühlt und über Leitung 13 einem Hochdruckabscheider 27 zugeführt. Im Hochdruckabscheider 27 erfolgt eine Phasentrennung in eine flüssige Methanol-Wasser Phase (Rohmethanol) sowie eine gasförmige Phase, welche im Wesentlichen nicht-reagiertes Synthesegas aufweist. Das nichtreagierte Synthesegas wird als Rückführgasstrom (Recycle-Gas) über Leitung 14 aus dem Hochdruckabscheider 27 abgezogen und einem Verdichter 28 (Rückführgas- oder Recycle-Gas-Kompressor) zugeführt, in dem das Rückführgas auf Reaktionsdruck verdichtet wird. Über Leitung 15 wird der Rückführgasstrom mit dem Synthesegasstrom aus Leitung 10 in Leitung 11 zusammengeführt, wodurch ein gemischtes Synthesegas als kombinierter Strom in Leitung 11 erhalten wird. Die Zusammensetzung des gemischten Synthesegases ergibt sich aus dem Verhältnis des frischen Synthesegasstroms in Leitung 10 und dem Rückführgasstrom in Leitung 15.

Das gemischte Synthesegas weist eine Stöchiometriezahl (SN_in) auf, die sich von der Stöchiometriezahl des frischen Synthesegases (SN_MUG) unterscheidet. Die Stöchiometriezahl des gemischten Synthesegases am Eingang des Katalysatorbettes wird durch gaschromatographische Analyse der Zusammensetzung des gemischten Synthesegases bestimmt, wie in Figur 2 gekennzeichnet (Gaschromatographie - GC). Das Verhältnis von Rückführgasstrom zu Synthesegasstrom, die Rezirkulationsrate (RR), wird gemäß den Zahlenbeispielen der Figuren 3a und 3b über einen Bereich von 0,194 bis 4,44 variiert.

Aus dem Rückführgas in Leitung 14 wird über Leitung 16 ein Spülgas (*purge gas*) abgezweigt und über Zwischenbehälter 29 aus dem Verfahren ausgeleitet (nicht gezeigt). Die Abzweigung des Spülgases verhindert das Ansammeln von inerten Bestandteilen innerhalb des Methanolsynthese-Kreislaufs.

Aus Hockdruckabscheider 27 wird Rohmethanol als flüssige Phase über Leitung 17 abgezogen und Niederdruckabscheider 30 zugeführt. Aus dem Rohmethanol in Niederdruckabscheider 30 werden weitere im Rohmethanol verbliebene, bis zu diesem Verfahrensschritt gelöste Gasbestandteile abgetrennt, die den Niederdruckabscheider 30 über Leitung 18 verlassen, und über den Zwischenbehälter 31 aus dem Verfahren ausgeleitet werden (nicht gezeigt).

Kondensiertes Rohmethanol wird aus dem Niederdruckabscheider 30 über Leitung 19 abgezogen, in Sammelbehälter 32 gesammelt und einer gaschromatographischen Analyse (GC) zur Ermittlung der gebildeten Nebenprodukte unterzogen. Die Ergebnisse sind im Detail in der tabellarischen Aufstellung der Figuren 3a und 3b aufgeführt.

Weitere Probennahmenstellen für gaschromatographische Analysen sind in Figur 2 entsprechend mit "GC" gekennzeichnet. Proben werden in regelmäßigen Abständen genommen, beispielsweise stündlich, um den Umsatz zu Methanol und die Selektivität der Reaktion zu verfolgen. Die verwendete gaschromatographische Methode ist von der Methode der International Methanol Producers & Consumers Associatin (IMPCA) abgeleitet, die beispielsweise unter http://www.methanol.org/wp-content/uploads/2016/07/IMPCA-Ref-Spec-08-December-2015.pdf beschrieben ist.

Die tabellarische Aufstellung der Figuren 3a und 3b zeigt die Versuchsergebnisse, die mit einer Versuchsanlage gemäß der vorstehend beschriebenen und in Figur 2 gezeigten erhalten wurden. Aufgeführt sind die erfindungsgemäßen Beispiele 1 bis 43 sowie die nicht erfindungsgemäßen Vergleichsbeispiele 101 bis 105. Gezeigt sind im Detail, in den Spalten von links nach rechts, wie folgt erläutert:

| Spalte (von links nach rechts) | Einheit | |
| --- | --- | --- |
| No. | | Beispiel Nr. 1 bis 43<br>Vergleichsbeispiele Nr. 101 bis 105 |
| p | barg | Druck im Reaktor (Synthesedruck) in bar gauge |
| SN_in | | Stöchiometriezahl des gemischten Synthesegases am Katalysatorbett-Eingang |
| $yCO_2$_in | Vol.-% | Anteil $CO_2$ im gemischten Synthesegas am Katalysatorbett-Eingang |
| yCO_in | Vol.-% | Anteil CO im gemischten Synthesegas am Katalysatorbett-Eingang |
| $XH_2$ | % | Umsatz Wasserstoff |

(fortgesetzt)

| Spalte (von links nach rechts) | Einheit | |
|---|---|---|
| Tmax | °C | Maximale Katalysatorbett-Temperatur |
| high Alc | ppm | Konzentration höherer Alkohole im Rohmethanol |
| Ketone | ppm | Konzentration Ketone im Rohmethanol |
| Ether | ppm | Konzentration Ether im Rohmethanol |
| Ester | ppm | Konzentration Ester im Rohmethanol |
| HC | ppm | Konzentration Kohlenwasserstoffe im Rohmethanol |
| Total | ppm | Gesamtkonzentration Nebenprodukte (höhere Alkohole, Ketone, Ether, Ester und Kohlenwasserstoffe) im Rohmethanol |

Die Kühltemperatur Tcool des Kühlmediums wurde über einen Bereich von ca. 200 °C bis ca. 250 °C variiert, um eine entsprechende maximale Katalysatorbett-Temperatur Tmax einzustellen. Das frische Synthesegas oder Frischgas wies eine Stöchiometriezahl SN_MUG zwischen 0,97 und 2,17 auf. Die Rezirkulationsrate RR wurde in Abhängigkeit von der Zusammensetzung (Stöchiometriezahl) des frischen Synthesegases SN_MUG und der gewünschten Stöchiometriezahl des gemischten Synthesegases am Katalysatorbett-Eingang SN_in zwischen ca. 0,2 und ca. 4,5 variiert. Die Raumgeschwindigkeit (*gas hourly space velocity*) wurde zwischen ca. 2200 und 16000 $Nm^3/(m^3h)$ variiert.

Alle Angaben in ppm sind auf die Masse bezogen (mg/kg).

Mit den genannten Einstellungen wurden Kohlenstoffdioxid-Umsätze $XCO_2$ von bis zu 97,0 %, Kohlenmonoxid-Umsätze XCO von bis zu 99,9 % sowie Gesamtkohlenstoffumsätze $XCO_x$ (Kohlendioxid und Kohlenmonoxid kumuliert) von bis zu 99,6 % erzielt.

Der Anteil des Wasserstoffs $yH_2\_in$ am Eingang des Katalysatorbettes ergibt sich aus der Stöchiometriezahl SN_in sowie $yCO_2\_in$ und yCO_in.

In den nicht erfindungsgemäßen Beispielen Nr. 101 bis 105 gemäß Fig. 3b (Vergleichsbeispiele) wurden jeweils Verunreinigungen in einer Gesamtkonzentration von deutlich oberhalb von 10000 ppm gefunden, nämlich zwischen 17900 und 31000 ppm. In allen fünf Vergleichsbeispielen liegt die Stöchiometriezahl des gemischten Synthesegases am Katalysatorbetteingang unter 0,80 und die Kohlenmonoxid-Konzentration im gemischten Synthesegas deutlich oberhalb von 20 Vol.-%.

Wird die Stöchiometriezahl des gemischten Synthesegases am Katalysatorbett-Eingang auf 0,80 oder mehr erhöht und die Kohlenmonoxid-Konzentration gleichzeitig auf 20 Vol.-% oder weniger gesenkt, wird entsprechend den Beispielen 1 bis 43 eine verminderte Nebenproduktbildung beobachtet, welche in Bezug auf die Gesamtheit der Nebenprodukte stets unter 10000 ppm liegt. Gleichzeitig wurde die maximale Katalysatorbett-Temperatur auf 280 °C oder weniger begrenzt. In den Beispielen 1 bis 43 weist die maximale Katalysatorbett-Temperatur eine Spannbreite von 205 °C bis 277 °C auf.

Wird die maximale Katalysatorbett-Temperatur auf 265 °C oder weniger begrenzt, sinkt die Konzentration der Nebenprodukte zuverlässig auf 5000 ppm oder weniger, was die Beispiele 1,2, 6-10, 13-16, 19-24, 28 und 33-43 zeigen.

Wird die maximale Katalyatorbett-Temperatur auf 250 °C oder weniger begrenzt, sinkt die Konzentration der Nebenprodukte weiter, auf 3500 ppm oder weniger, was die Beispiele 8, 9, 14-16, 19-23, 35-38 und 41-43 zeigen.

Selbst vergleichsweise niedrige Stöchiometriezahlen von 0,80 bis 2,20 für das gemischte Synthesegas am Katalysatorbett-Eingang (SN_in) führen unter Einhaltung der erfindungsgemäßen Bedingungen zu weniger als 10000 ppm Verunreinigungen, was durch die Beispiele 9-23 und 34-43 gezeigt wird. In diesem Zusammenhang ist es besonders günstig, wenn der Anteil an CO im gemischten Synthesegas bei 9,0 bis 13,0 Vol.-% liegt, da dann trotz der niedrigen Stöchiometriezahl zuverlässig ein Wasserstoff-Umsatz von deutlich über 80 %, hier von 86,8 bis 98,7 %, erzielt wird, wie durch die Beispiele 9-12, 14-17, 19-21 und 23 gezeigt.

Liegt die Stöchiometriezahl des gemischten Synthesegases am Katalysatorbett-Eingang bei 2,0 oder höher, liegt die Konzentration der Verunreinigungen zuverlässig bei 5000 ppm oder weniger, wie durch die Beispiele 1-9, 15, 16 und 24-36 gezeigt.

Das erfindungsgemäße Verfahren eignet sich insbesondere für den Einsatz von Synthesegasen mit hohem Kohlendioxid-Gehalt. Liegt der Kohlendioxid-Gehalt im gemischten Synthesegas bei 25 Vol.-% oder mehr, werden zuverlässig weniger als 1000 ppm Nebenprodukte gebildet, wie durch die Beispiele 34-43 gezeigt.

Ausführungsformen der Erfindung werden unter Bezugnahme auf verschiedene Arten von Gegenständen beschrieben. Insbesondere werden bestimmte Ausführungsformen unter Bezugnahme auf Ansprüche des Verfahrenstyps beschrieben, während andere Ausführungsformen unter Bezugnahme auf die Ansprüche des Vorrichtungstyps beschrieben werden. Ein Fachmann wird jedoch aus der obigen und der folgenden Beschreibung entnehmen, dass, außer anders angegeben, zusätzlich zu irgendeiner Kombination von Merkmalen, die zu einer Art von Anspruchstyp gehören, auch jede Kombination von Merkmalen in Bezug auf verschiedene Arten von Gegenständen oder Anspruchstypen in Betracht gezogen werden kann. Merkmale können kombiniert werden um synergetische Effekte zu erzielen, welche über die einfache Summierung der technischen Merkmale hinausgehen.

Während die Erfindung im Detail in der Zeichnung und der vorhergehenden Beschreibung dargestellt und beschrieben wurde, sollen eine solche Darstellung und Beschreibung als veranschaulichend oder beispielhaft und nicht einschränkend betrachtet werden. Die Erfindung ist nicht auf die offenbarten Ausführungsformen beschränkt. Andere Variationen der offenbarten Ausführungsformen können von einem Fachmann auf dem Gebiet der beanspruchten Erfindung aus einem Studium der Zeichnung, der Offenbarung und der abhängigen Ansprüche verstanden und ausgeführt werden.

In den Ansprüchen schließt das Wort "aufweisend" oder "umfassend" weitere Elemente oder Schritte nicht aus, und der unbestimmte Artikel "ein" oder "eine" schließt eine Mehrzahl nicht aus. Bezugszeichen in den Ansprüchen sollten nicht so ausgelegt werden, dass sie den Umfang der Ansprüche einschränken.

**Bezugzeichenliste**

| | |
|---|---|
| 1 | Verfahren, Versuchsanlage |
| 10-19 | Leitung |
| 20 | Mischstation |
| 21 | Reaktor |
| 22, 26, 28 | Wärmeaustauscher |
| 23 | Reaktionsrohr |
| 24 | Kühlmantel |
| 25 | Katalysatorbett |
| 27 | Hochdruckabscheider |
| 29, 31 | Zwischenbehälter |
| 30 | Niederdruckabscheider |
| 32 | Sammelbehälter |

**Patentansprüche**

1. Verfahren zur Herstellung von Methanol, wobei das Verfahren folgende Prozessschritte umfasst:

   a. Bereitstellen eines Kohlenstoffoxide und Wasserstoff aufweisenden Synthesegases;
   b. Leiten des Synthesegases bei erhöhtem Druck und erhöhter Temperatur durch ein Katalysatorbett eines Methanolsynthese-Katalysators zur Umsetzung des Synthesegases zu Methanol, wobei ein Rohmethanol und nicht-reagiertes Synthesegas umfassender Produktstrom erhalten wird;
   c. Kühlen des Produktstroms zur Kondensation und Abtrennung von zumindest Methanol und Wasser aufweisendem Rohmethanol aus dem gekühlten Produktstrom;
   d. Zurückführen zumindest eines Teils des nicht-reagierten Synthesegases zum Eingang des Katalysatorbetts, wobei das nicht reagierte Synthesegas mit dem Synthesegas zusammengeführt wird, wobei ein gemischtes Synthesegas erhalten wird, und Leiten des gemischten Synthesegases bei erhöhtem Druck und erhöhter Temperatur durch das Katalysatorbett des Methanolsynthese-Katalysators zur Umsetzung des gemischten Synthesegases zu Methanol,

   **dadurch gekennzeichnet, dass**

   das gemischte Synthesegas am Eingang des Katalysatorbetts eine Stöchiometriezahl SN von 0,80 bis 2,20 aufweist, wobei gilt

$$SN = \frac{n(H_2) - n(CO_2)}{n(CO) + n(CO_2)} \, ,$$

mit n in [mol],
das Katalysatorbett bei der Umsetzung des gemischten Synthesegases zu Methanol eine maximale Katalysatorbett-Temperatur von $\leq 280\,°C$ aufweist, und das gemischte Synthesegas am Eingang des Katalysatorbettes eine Kohlenmonoxid-Konzentration von 9,0 bis 13,0 Vol.-% aufweist.

2. Verfahren nach Anspruch 1, **dadurch gekennzeichnet, dass** das Katalysatorbett bei der Umsetzung des gemischten Synthesegases zu Methanol eine maximale Katalysatorbett-Temperatur von $\leq 265\,°C$ aufweist.

3. Verfahren nach Anspruch 1, **dadurch gekennzeichnet, dass** das Katalysatorbett bei der Umsetzung des gemischten Synthesegases zu Methanol eine maximale Katalysatorbett-Temperatur von $205\,°C$ bis $280\,°C$ aufweist.

4. Verfahren nach Anspruch 1 oder 2, **dadurch gekennzeichnet, dass** das Katalysatorbett bei der Umsetzung des gemischten Synthesegases zu Methanol eine maximale Katalysatorbett-Temperatur von $205\,°C$ bis $265\,°C$ aufweist.

5. Verfahren nach einem der vorherigen Ansprüche, **dadurch gekennzeichnet, dass** das Synthesegas eine Stöchiometriezahl SN von 1,0 bis 2,85 aufweist, vorzugsweise eine Stöchiometriezahl SN von 1,0 bis 2,30 aufweist.

6. Verfahren nach einem der vorherigen Ansprüche, **dadurch gekennzeichnet, dass** das gemischte Synthesegas am Eingang des Katalysatorbettes eine Kohlendioxid-Konzentration von $\geq 20,0$ Vol.-% aufweist.

7. Verfahren nach einem der vorhergehenden Ansprüche, **dadurch gekennzeichnet, dass** das Katalysatorbett auf mehrere in Serie angeordnete Katalysatorbettstufen aufgeteilt ist, wobei Schritt c) nach jeder der Katalysatorbettstufen durchgeführt wird.

8. Verfahren nach einem der vorherigen Ansprüche, **dadurch gekennzeichnet, dass** die Umsetzung des Synthesegases zu Methanol im Katalysatorbett bei einer Kühltemperatur des verwendeten Kühlmediums von $190\,°C$ bis $250\,°C$ erfolgt.

**Claims**

1. Process for producing methanol, wherein the process comprises the following process steps:

   a. providing a synthesis gas including carbon oxides and hydrogen;
   b. passing the synthesis gas at elevated pressure and elevated temperature through a catalyst bed of a methanol synthesis catalyst for conversion of the synthesis gas to methanol to obtain a product stream comprising crude methanol and unreacted synthesis gas;
   c. cooling the product stream for condensation and separation of crude methanol comprising at least methanol and water from the cooled product stream;
   d. recycling at least a portion of the unreacted synthesis gas to the catalyst bed inlet, wherein the unreacted synthesis gas is combined with the synthesis gas to obtain a mixed synthesis gas, and passing the mixed synthesis gas at elevated pressure and elevated temperature through the catalyst bed of the methanol synthesis catalyst for conversion of the mixed synthesis gas to methanol,

   **characterized in that**

   the mixed synthesis gas at the catalyst bed inlet has a stoichiometry number SN of 0.80 to 2.20, where

$$SN = \frac{n(H_2) - n(CO_2)}{n(CO) + n(CO_2)} \, ,$$

   with n in [mol],
   the catalyst bed in the conversion of the mixed synthesis gas to methanol has a maximum catalyst bed

temperature of $\leq$ 280°C, and the mixed synthesis gas at the catalyst bed inlet has a carbon monoxide concentration of 9.0% to 13.0% by volume.

2. Process according to Claim 1, **characterized in that** the catalyst bed in the conversion of the mixed synthesis gas to methanol has a maximum catalyst bed temperature of $\leq$ 265°C.

3. Process according to Claim 1, **characterized in that** the catalyst bed in the conversion of the mixed synthesis gas to methanol has a maximum catalyst bed temperature of 205°C to 280°C.

4. Process according to Claim 1 or 2, **characterized in that** the catalyst bed in the conversion of the mixed synthesis gas to methanol has a maximum catalyst bed temperature of 205°C to 265°C.

5. Process according to any of the preceding claims, **characterized in that** the synthesis gas has a stoichiometry number SN of 1.0 to 2.85, preferably a stoichiometry number SN of 1.0 to 2.30.

6. Process according to any of the preceding claims, **characterized in that** the mixed synthesis gas at the catalyst bed inlet has a carbon dioxide concentration of $\geq$ 20.0% by volume.

7. Process according to any of the preceding claims, **characterized in that** the catalyst bed is divided into a multitude of catalyst bed stages arranged in series, wherein step c) is conducted downstream of each of the catalyst bed stages.

8. Process according to any of the preceding claims, **characterized in that** the synthesis gas is converted to methanol in the catalyst bed at a cooling temperature of the cooling medium used of 190°C to 250°C.

## Revendications

1. Procédé pour la production de méthanol, le procédé comprenant les étapes de processus suivantes :

   a. mise à disposition d'un gaz de synthèse comportant des oxydes de carbone et de l'hydrogène ;
   b. passage du gaz de synthèse sous pression et à température élevées à travers un lit de catalyseur d'un catalyseur de synthèse de méthanol pour la conversion du gaz de synthèse en méthanol, avec obtention d'un courant de produit comprenant du méthanol brut et du gaz de synthèse n'ayant pas réagi ;
   c. refroidissement du courant de produit pour la condensation et la séparation de méthanol brut comportant au moins du méthanol et de l'eau à partir du courant de produit refroidi ;
   d. renvoi d'au moins une partie du gaz de synthèse n'ayant pas réagi à l'entrée du lit de catalyseur, le gaz de synthèse n'ayant pas réagi étant réuni avec le gaz de synthèse, avec obtention d'un gaz de synthèse mélangé, et passage du gaz de synthèse mélangé sous pression élevée et à température élevée à travers le lit de catalyseur du catalyseur de synthèse de méthanol, pour la conversion du gaz de synthèse mélangé en méthanol,

   **caractérisée en ce que**

   le gaz de synthèse mélangé présente à l'entrée du lit de catalyseur un indice stœchiométrique SN de 0,80 à 2,20, où il est défini que

   $$SN = \frac{n(H_2) - n(CO_2)}{n(CO) + n(CO_2)} \, ,$$

   avec n en [*moles*],
   le lit de catalyseur présente lors de la conversion du gaz de synthèse mélangé en méthanol une température maximale de lit de catalyseur de $\leq$ 280 °C, et le gaz de synthèse mélangé présente à l'entrée du lit de catalyseur une teneur en monoxyde de carbone de 9,0 à 13,0 % en volume.

2. Procédé selon la revendication 1, **caractérisé en ce que** le lit de catalyseur présente lors de la conversion du gaz de synthèse mélangé en méthanol une température maximale de lit de catalyseur de $\leq$ 265 °C.

3. Procédé selon la revendication 1, **caractérisé en ce que** le lit de catalyseur présente lors de la conversion du gaz de synthèse mélangé en méthanol une température maximale de lit de catalyseur de 205 °C à 280 °C.

4. Procédé selon la revendication 1 ou 2, **caractérisé en ce que** le lit de catalyseur présente lors de la conversion du gaz de synthèse mélangé en méthanol une température maximale de lit de catalyseur de 205 °C à 265 °C.

5. Procédé selon l'une quelconque des revendications précédentes, **caractérisé en ce que** le gaz de synthèse présente un indice stœchiométrique SN de 1,0 à 2,85, de préférence un indice stœchiométrique SN de 1,0 à 2,30.

6. Procédé selon l'une quelconque des revendications précédentes, **caractérisé en ce que** le gaz de synthèse mélangé présente à l'entrée du lit catalytique une teneur en dioxyde de carbone de $\geq$ 20,0 % en volume .

7. Procédé selon l'une quelconque des revendications précédentes, **caractérisé en ce que** le lit de catalyseur est divisé en plusieurs étages de lit de catalyseur disposés en série, l'étape c) étant effectuée après chacun des étages de lit de catalyseur.

8. Procédé selon l'une quelconque des revendications précédentes, **caractérisé en ce que** la conversion du gaz de synthèse en méthanol s'effectue dans le lit de catalyseur à une température de refroidissement du milieu de refroidissement utilisé de 190°C à 250°C.

**Fig. 1**

Fig. 2

| No. | p / barg | SN_in | yCO₂_in / vol% | yCO_in / vol% | X_H₂ / % | Tmax / °C | high Alc / ppm | Ketone / ppm | Ether / ppm | Ester / ppm | HC / ppm | Total / ppm |
|---|---|---|---|---|---|---|---|---|---|---|---|---|
| 1 | 73,2 | 9,57 | 1,87 | 6,71 | 95,6 | 262 | 1150 | 39,9 | 1200 | 433 | 66,0 | 2890 |
| 2 | 73,2 | 9,44 | 1,92 | 6,77 | 95,2 | 263 | 1150 | 39,1 | 1170 | 416 | 64,3 | 2830 |
| 3 | 73,2 | 7,08 | 2,73 | 8,52 | 93,8 | 269 | 1560 | 53,5 | 1300 | 562 | 59,2 | 3540 |
| 4 | 80,0 | 4,62 | 6,46 | 7,88 | 83,9 | 277 | 1620 | 62,6 | 845 | 959 | 50,1 | 3540 |
| 5 | 85,0 | 3,7 | 7,85 | 9,52 | 81,4 | 268 | 985 | 65,5 | 448 | 1200 | 56,8 | 2750 |
| 6 | 85,0 | 3,72 | 7,85 | 9,49 | 81,3 | 263 | 694 | 52,6 | 243 | 1260 | 37,8 | 2290 |
| 7 | 85,0 | 3,72 | 7,83 | 9,5 | 81,3 | 260 | 638 | 49,1 | 210 | 1220 | 34,3 | 2150 |
| 8 | 70,0 | 2,63 | 11,4 | 9,79 | 90,2 | 236 | 239 | 13,9 | 72,5 | 1950 | 11,7 | 2280 |
| 9 | 70,0 | 2,03 | 13,4 | 10,9 | 92,2 | 235 | 239 | 13,9 | 72,5 | 1950 | 11,7 | 2280 |
| 10 | 50,0 | 1,75 | 14,2 | 12,9 | 89,4 | 264 | 2160 | 104 | 446 | 1570 | 51,1 | 4330 |
| 11 | 80,0 | 1,76 | 13,6 | 9,56 | 98,2 | 273 | 3100 | 126 | 939 | 1810 | 80,1 | 6050 |
| 12 | 79,9 | 1,45 | 14,5 | 9,05 | 98,7 | 267 | 2850 | 125 | 866 | 1890 | 71,3 | 5800 |
| 13 | 65,0 | 1,95 | 13,0 | 14,8 | 77,5 | 254 | 936 | 82,5 | 182 | 2740 | 53,3 | 3990 |
| 14 | 70,0 | 1,97 | 13,5 | 11,4 | 91,2 | 233 | 180 | 20,8 | 56,1 | 2220 | 8,78 | 2490 |
| 15 | 80,0 | 2,09 | 13,2 | 12,5 | 86,8 | 243 | 142 | 21,5 | 43,7 | 2680 | 5,19 | 2900 |
| 16 | 70,0 | 2,01 | 13,1 | 12,3 | 88,9 | 231 | 896 | 73,7 | 208 | 2030 | 52,7 | 3260 |
| 17 | 65,0 | 1,97 | 13,5 | 11,6 | 91,0 | 268 | 1820 | 93,1 | 417 | 1850 | 51,3 | 4230 |
| 18 | 50,0 | 1,88 | 12,4 | 16,7 | 66,8 | 272 | 5510 | 237 | 763 | 2970 | 123 | 9600 |
| 19 | 65,0 | 1,98 | 13,1 | 13,0 | 86,2 | 243 | 470 | 55,2 | 105 | 2260 | 25,9 | 2910 |
| 20 | 50,0 | 1,76 | 14,6 | 9,20 | 96,7 | 235 | 402 | 41,8 | 128 | 1650 | 23,9 | 2240 |
| 21 | 70,0 | 1,88 | 13,8 | 12,9 | 87,0 | 231 | 196 | 31,2 | 48,0 | 2230 | 9,84 | 2520 |
| 22 | 30,1 | 1,7 | 13,8 | 16,0 | 73,3 | 239 | 414 | 46,0 | 127 | 1600 | 24,3 | 2210 |
| 23 | 70,0 | 1,98 | 13,9 | 10,6 | 91,9 | 235 | 229 | 22,2 | 64,1 | 1970 | 10,1 | 2290 |
| 24 | 73,2 | 9,02 | 1,81 | 7,20 | 96,9 | 260 | 1090 | 42,9 | 400 | 490 | 62,4 | 2080 |
| 25 | 95,0 | 4,26 | 6,38 | 5,7 | 83,2 | 267 | 1120 | 42,5 | 326 | 879 | 52,7 | 2420 |
| 26 | 82,3 | 5,03 | 5,58 | 6,29 | 92,9 | 267 | 1290 | 51,1 | 344 | 770 | 67,4 | 2520 |
| 27 | 82,5 | 6,7 | 4,40 | 5,65 | 92,5 | 266 | 928 | 33,8 | 301 | 597 | 45,7 | 1900 |
| 28 | 95,0 | 4,92 | 5,23 | 5,07 | 84,5 | 264 | 2210 | 67,0 | 358 | 1850 | 34,2 | 4520 |

Fig. 3a

| No. | p / barg | SN_in | yCO$_2$_in / vol% | yCO_in / vol% | X_H$_2$ / % | Tmax / °C | high Alc / ppm | Ketone / ppm | Ether / ppm | Ester / ppm | HC / ppm | Total / ppm |
|---|---|---|---|---|---|---|---|---|---|---|---|---|
| 29 | 95,0 | 4,91 | 5,68 | 5,73 | 89,3 | 267 | 1090 | 40,9 | 330 | 749 | 53,1 | 2260 |
| 30 | 82,3 | 5,3 | 5,17 | 6,11 | 93,3 | 266 | 859 | 32,0 | 287 | 726 | 38,0 | 1940 |
| 31 | 82,5 | 5,86 | 5,04 | 6,35 | 92,5 | 268 | 898 | 33,7 | 303 | 708 | 42,6 | 1990 |
| 32 | 60,0 | 3,26 | 7,06 | 14,3 | 76,3 | 266 | 2070 | 132 | 349 | 2070 | 44,2 | 4660 |
| 33 | 80,0 | 2,53 | 16,5 | 1,97 | 95,0 | 255 | 1720 | 55,2 | 182 | 2890 | 16,0 | 4870 |
| 34 | 89,8 | 2,06 | 23,7 | 0,498 | 41,2 | 257 | 98,7 | 1,63 | 44,3 | 586 | 0 | 731 |
| 35 | 89,5 | 2,04 | 23,9 | 0,417 | 33,5 | 234 | 38,4 | 2,72 | 13,7 | 680 | 0 | 735 |
| 36 | 90,0 | 2,06 | 23,8 | 0,375 | 29,3 | 218 | 21,7 | 3,37 | 6,73 | 931 | 0 | 963 |
| 37 | 90,0 | 0,941 | 33,0 | 0,487 | 31,3 | 218 | 12,0 | 4,71 | 4,82 | 889 | 0 | 910 |
| 38 | 90,0 | 0,916 | 33,2 | 0,508 | 40,9 | 234 | 14,4 | 5,03 | 4,50 | 878 | 0 | 902 |
| 39 | 90,0 | 0,891 | 33,5 | 0,581 | 45,5 | 256 | 118 | 5,99 | 19,0 | 766 | 0 | 909 |
| 40 | 89,3 | 1,54 | 27,3 | 0,489 | 44,1 | 257 | 111 | 5,09 | 18,1 | 747 | 0 | 882 |
| 41 | 89,8 | 1,59 | 26,9 | 0,48 | 36 | 234 | 49,8 | 6,47 | 10,0 | 794 | 0 | 860 |
| 42 | 89,8 | 1,64 | 26,6 | 0,464 | 26,8 | 218 | 26,1 | 6,80 | 5,42 | 935 | 0 | 974 |
| 43 | 90,0 | 1,46 | 25,6 | 3,26 | 54,2 | 218 | 22,1 | 5,70 | 4,60 | 826 | 0 | 858 |
| 101 | 70,0 | 0,494 | 12,1 | 41,1 | 97,8 | 255 | 11800 | 445 | 1100 | 17500 | 130 | 31000 |
| 102 | 70,0 | 0,371 | 11,4 | 47,4 | 97,6 | 242 | 9350 | 318 | 872 | 14400 | 87,3 | 25000 |
| 103 | 50,2 | 0,713 | 10,6 | 39,4 | 93,2 | 253 | 11700 | 311 | 1070 | 10900 | 131 | 24000 |
| 104 | 50,0 | 0,505 | 10,5 | 45,0 | 95,5 | 234 | 6840 | 237 | 675 | 10000 | 63,3 | 17900 |
| 105 | 70,0 | 0,338 | 13,7 | 45,2 | 97,4 | 242 | 7850 | 281 | 758 | 15100 | 73,3 | 24100 |

Fig. 3b

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2020048809 A

**In der Beschreibung aufgeführte Nicht-Patentliteratur**

- *Chemical Engineering Science*, 1994, vol. 49 (2), 209-221
- *Chemical Engineering Science*, 1986, vol. 41 (11), 2883-2890
- *Chemical Engineering Science*, 1988, vol. 43 (12), 3185-3195
- *Chemical Engineering Science*, 1991, vol. 46 (11), 2809-2813
- *Chemical Engineering Science*, 2001, vol. 56 (14), 4321-4329
- *Zh. Fiz. Khim*, 1949, vol. 23 (1), 342-360
- *Archiv für das Eisenhüttenwesen*, 1964, vol. 35 (2), 91-108
- The properties of gases and liquids. McGraw-Hill, 2001, vol. 5
- *Chem. Eng. Prog.*, 1952, vol. 48, 89-94
- *Chemical Engineering Science*, 1972, vol. 27 (6), 1197-1203
- **G.C.CHINCHEN et al.** *Appl. Catal.*, 1988, vol. 36, 1-65